# EUROPÄISCHE PATENTSCHRIFT

(11) **EP 2 498 833 B1**
(45) Veröffentlichungstag und Bekanntmachung des Hinweises auf die Patenterteilung: **08.01.2014**
(21) Anmeldenummer: 10778649.3
(22) Anmeldetag: 12.11.2010
(51) Int. Cl.: A61L 29/06, A61L 29/16

(54) **VERWENDUNG POLYMERER ODER OLIGOMERER WIRKSTOFFE FÜR MEDIZINISCHE ARTIKEL**
USE OF POLYMERIC OR OLIGOMERIC ACTIVE INGREDIENTS FOR MEDICAL ARTICLES
UTILISATION D'AGENTS POLYMÉRIQUES OU OLIGOMÉRIQUES POUR DES ARTICLES MÉDICAUX

(30) Priorität: 12.11.2009 DE 102009052721
(43) Veröffentlichungstag der Anmeldung: 19.09.2012
(73) Patentinhaber: B. Braun Melsungen AG, 34212 Melsungen (DE)
(72) Erfinder: WEIß, André, 34302 Guxhagen (DE); RIEMANN, Thomas, 37247 Großalmerode (DE); SIPPEL, Martin, 34212 Melsungen (DE)
(74) Vertreter: von Kreisler Selting Werner
(86) Internationale Anmeldenummer: PCT/EP2010/067411
(87) Internationale Veröffentlichungsnummer: WO 2011/058148

(56) Entgegenhaltungen:
- WO-A2-2009/009814
- WO-A2-2009/080239
- US-A1- 2002 120 333

## Beschreibung

Die Erfindung betrifft die Verwendung polymerer oder oligomerer Wirkstoffe mit biozider Wirkung als Additiv in einer Zusammensetzung für medizinische Artikel.

Darüber hinaus betrifft die Erfindung ein Verfahren zur Herstellung eines medizinischen Artikels sowie einen medizinischen Artikel.

Medizinische Artikel bzw. Gegenstände, die in den Körper eines Patienten eingeführt werden, beispielsweise intravasale Katheter, Beatmungsschläuche oder Stents müssen eine möglichst glatte Oberfläche haben, um Beschwerden beim Patienten als auch Ablagerungen auf den Oberflächen - zu minimieren. Hergestellt werden diese medizinischen Artikel sowie deren Verpackung oft durch Verfahren der Kunststofftechnik, beispielsweise Formpress-, Spritzguss-, Tiefzieh- und Extrusionsverfahren aus einem Kunststoff, wobei versucht wird, möglichst glatte Oberflächen zu erzielen.

Zur Vermeidung von Infektionen ist es vorteilhaft die medizinischen Artikel mit antimikrobiell wirkenden Mitteln ausgerüstet sein. An die biozide Ausrüstung der medizinischen Artikel werden hohe Anforderungen gestellt, da die Artikel mit Körpergewebe und Körperflüssigkeit in Kontakt kommen. Beispielsweise stellen Katheter, die durch die Hautoberfläche in Arterien oder Venen eingeführt werden, aber auch Wund- oder Thoraxdränageschläuche, häufige Infektionsquellen dar. Insbesondere besteht bei Patienten, die Langzeit-Urinkatheter benötigen, die Gefahr von Harnwegsinfektionen, die zu einer Bakterien- oder einer chronischen Pyelonephritis führen können.

Im medizinischen Bereich spielen insbesondere die zentralen Venenkatheter eine zunehmend größere Rolle bei medizinischen Behandlungen und chirurgischen Eingriffen. Zentrale Venenkatheter werden immer häufiger im Rahmen der Intensivmedizin, aber auch bei Anwendungen, z.B. bei Knochenmarks- und Organtransplantationen, Hämodialyse oder Cardiothoraxchirurgie, eingesetzt.

Ein ähnliches Infektionsrisiko ist aber auch bei allen Vorrichtungen gegeben, welche die Katheter beispielsweise mit Infusionsbehältnissen außerhalb des Körpers verbinden, beispielsweise Verbindungsstücke, T-Stücke, Kupplungen, Filter, Überleitungssysteme, Ventile, Spritzen und Mehrweg-Hähne. All diese Gegenstände werden für die Zwecke dieser Beschreibung und der Patentansprüche als "medizinische Artikel" bezeichnet.

Für die medizinischen Artikel, insbesondere die Katheter, müssen aber nicht nur die hohen Anforderungen an eine glatte Oberfläche beispielsweise zur Vermeidung bzw. Verringerung von Thrombozytenapposition und Biofilmbildung und an die biozide Ausrüstung, die ein Wachsen von Mikroben auf der Oberfläche verhindern oder die Mikroben ganz abtöten sollen, gewährleistet sein, sondern es muss auch sichergestellt werden, dass die biozide Ausrüstung der medizinischen Gegenstände die Materialeigenschaften der medizinischen Artikel nicht negativ beeinflusst. Darüber hinaus muss gewährleistet werden, dass die medizinischen Artikel, insbesondere dann, wenn sie mit Flüssigkeit in Kontakt kommen, einerseits eine hohe biozide Wirksamkeit aufweisen, aber andererseits auch nicht an die Flüssigkeit abgegeben werden, um eine Anreicherung der bioziden Wirkstoffe im Körper zu vermeiden. Die Abgabe eines bioziden Wirkstoffs aus einem medizinischen Artikel bei Flüssigkeitskontakt wird auch als "Leaching" bezeichnet.

Aus der EP 0 229 862 sind medizinische Artikel aus Polyurethan bekannt, auf deren Oberfläche ein antimikrobielles Mittel aufgebracht ist. Aus der EP 0 379 269 sind ebenfalls medizinische Artikel, insbesondere Schläuche, bekannt, welche aus einem thermoplastischen Polymer geformt sind und als antimikrobiell wirkendes Mittel Chlorhexidin enthalten. Diese Artikel werden hergestellt, indem zunächst eine Mischung aus Chlorhexidin und Kunststoffgranulate eines thermoplastischen Polymers bereitgestellt wird, die zu einer Schmelze verarbeitet wird, in der das Chlorhexidin gleichmäßig verteilt ist, wobei die Schmelze durch eine Matrix extrudiert wird, um den medizinischen Artikel zu formen. Der Einsatz von bioziden Mitteln auf Basis von Biguaniden, wie beispielsweise Chlorhexidin oder Polyhexamethylenbiguanid, ist jedoch nicht immer zufriedenstellend. Insbesondere besteht ein Verbesserungsbedarf im Hinblick auf die Glattheit der Oberfläche der medizinischen Artikel als auch im Hinblick auf die Reduzierung oder Steuerung des "Leaching"-Effekts.

WO 2009/009814 A2 offenbart einen polymeren Dentalwerkstoff, umfassend einen silikatischen Füllstoff, der mit einem polymeren Guanidinderivat auf Basis eines Alkylendiamins und/oder eines Oxyalkylendiamins modifiziert ist. Explizit offenbart wird das polymere Guanidinderivat Poly-[2-(2-ethoxy-ethoxyethyl)-guanidium-hydrochlorid], welches jedoch hinsichtlich der antimikrobiellen Wirksamkeit als auch hinsichtlich der Biokompatibilität für Anwendungen von Medizinprodukten der Klasse 3 mit direktem Kontakt zum Blutkreislauf keine zufriedenstellende Ergebnisse lieferte.

Darüber hinaus war es Aufgabe der vorliegenden Erfindung, medizinische Artikel zur Verfügung zu stellen, die mit neuartigen bioziden Wirkstoffen ausgerüstet sind, die auch im Hinblick auf eine Resistenz-Entwicklung der Bakterienstämme bezüglich herkömmlicher antimikrobieller Mittel hochwirksam sind.

An biozide Wirkstoffe, die für die Herstellung von medizinischen Artikeln vorgesehen sind, werden hohe Anforderungen gestellt. Zum Einen müssen die bioziden Wirkstoffe unter den Bedingungen der Verarbeitungstechnik, nämlich der thermoplastischen Formgebung, wie Extrusion oder Spritzguss und den dabei auftretenden Temperaturen und Drücken hinreichend erweichen, dürfen sich nicht zersetzen und müssen zudem mit den übrigen Kunststoffbestandteilen des medizinischen Artikel kompatibel sein.

Insbesondere für medizinische Artikel, die im direkten Kontakt mit dem Blutkreislauf stehen, werden zudem hohe Anforderungen an die Biokompatibilität, wobei Faktoren, wie die Zytotoxizität, Hämolyse oder auch allergische Reaktionen eine Rolle spielen, gestellt. Darüber hinaus soll vermieden werden, dass die bioziden Wirkstoffe an das Blut abgegeben werden, es aber gleichsam den medizinischen Artikel mit einer hohen antimikrobiellen Wirksamkeit ausstattet.

Die Aufgabe der vorliegenden Erfindung wird durch die Verwendung neuartiger polymerer oder oligomerer Wirkstoffe als Additive zu Werkstoffen für medizinische Artikel gelöst.

Gegenstand der vorliegenden Erfindung ist daher die Verwendung eines polymeren oder oligomeren Wirkstoffs mit biozider Wirkung, welcher erhältlich ist durch Polykondensation eines Guanidin-Säureadditionssalzes mit einem Amingemisch, welches wenigstens ein Diamin und/oder ein Triamin enthält, wobei wenigstens ein Amin ausgewählt ist aus der Gruppe, die aus
i) Diamin, das wenigstens einen cycloaliphatischen Rest aufweist, und
ii) Dialkylentriamin besteht,
als Additiv in einer Zusammensetzung für medizinische Artikel.

Es hat sich gezeigt, dass bei Verwendung der genannten polymeren oder oligomeren Wirkstoffe in Zusammensetzungen, die Kunststoffe, insbesondere thermoplastische Polymere, enthalten, wobei die Kunststoffe für die Herstellung medizinischer Artikel eingesetzt wird, sehr glatte Oberflächen des Kunststoffs gewährleistet werden können, welche sogar jene des Kunststoffs ohne den Zusatz der polymeren oder oligomeren Wirkstoffe übertreffen kann. Neben der hervorragenden bioziden Wirksamkeit, die die polymeren oder oligomeren Wirkstoffe den Zusammensetzungen für die medizinischen Artikel verleihen, hat sich auch überraschend herausgestellt, dass die polymeren oder oligomeren Wirkstoffe, die in Zusammensetzungen, umfassend Kuststoffe, insbesondere thermoplastische Polymere, in Abhängigkeit von der Auswahl derselben ein gut steuerbares Freisetzungsverhalten ("Leaching"-Effekt) aufweisen. Die Steuerbarkeit reicht von keiner Freisetzung bis zu Freisetzungsraten von mehreren mg/m²/h.

Es hat sich zudem überraschend gezeigt, dass die erfindungsgemäß einzusetzenden polymeren oder oligomeren Wirkstoffe eine gute Biokompatibilität besitzen, eine hohe antimikrobielle Wirksamkeit gegen eine Vielzahl von Keimen aufweisen und zudem lediglich eine geringe Beeinflussung der thermoplastischen Polymere, die für die Herstellung der medizinischen Artikel benötigt werden, hinsichtlich Festigkeit, Glanz sowie Haltbarkeit zeigen.

Die erfindungsgemäß verwendeten polymeren oder oligomeren Wirkstoffe können sowohl als Homopolymer als auch als Copolymer vorliegen. Vorteilhaft ist dabei, wenn das Guanidin-Säureadditionssalz Guanidiniumhydrochlorid ist. Es sind aber auch weitere Guanidin-Säureadditionssalze, auf Basis anorganischer oder organischer Säuren ebenfalls geeignet. Beispielsweise die Hydroxide, Hydrogensulfate sowie Acetate.

Die erfindungsgemäß verwendeten polymeren oder oligomeren Wirkstoffe liegen bevorzugt als Hydroxidsalz vor. Diese können beispielsweise durch basischen Anionenaustausch aus den entsprechenden Halogeniden, z.B. Chloriden, erhalten werden.

Die polymeren oder oligomeren Wirkstoffe mit biozider Wirkung sind erhältlich durch Polykondensation eines Guanidin-Säureadditionssalzes mit einem Amingemisch, welches wenigstens ein Diamin und/oder ein Triamin enthält, wobei wenigstens ein Amin ausgewählt ist aus der Gruppe, die aus
i) Diamin, das wenigstens einen cycloaliphatischen Rest aufweist, und
ii) Dialkylentriamin besteht.

Die durch die Polykondensation erhältlichen polymeren oder oligomeren Wirkstoffe weisen hierbei bevorzugt eine Polyguanidinstruktur oder, insbesondere im Falle des Einsatzes von Dialkylentriaminen, beispielsweise Diethylentriamin, eine Poly(iminoimidazol)-Struktur auf.

In einer bevorzugten Ausgestaltung der vorliegenden Erfindung umfasst das Amingemisch die Komponente i) (Diamin, das wenigstens einen cycloaliphatischen Rest aufweist) und/oder die Komponente ii) (Dialkylentriamin) in einer Menge von wenigstens 10 mol-%, bevorzugt mindestens 25 mol-%, weiter bevorzugt mindestens 45 mol-%, insbesondere mindestens 85 mol-%, im Speziellen mindestens 95 mol-%, jeweils bezogen auf das gesamte Amingemisch.

Bevorzugt umfasst das Amingemisch zusätzlich ein Alkylendiamin, das besonders bevorzugt eine Verbindung der allgemeinen Formel

NH₂(CH₂)ₙNH₂ in welcher n eine ganze Zahl zwischen 2 und 10, bevorzugt 4 oder 6, ist. Bevorzugt einzusetzende Alkylendiamine tragen endständige Aminogruppen. Speziell bevorzugt ist das Hexamethylendiamin (Hexan-1,6-diamin). Das Alkylendiamin kann in der Polykondensationsreaktion in Mischung mit weiteren Diaminen oder Triaminen, wobei wenigstens ein Amin ausgewählt ist aus der Gruppe, die aus

i) Diamin, das wenigstens einen cycloaliphatischen Rest aufweist, und
ii) Dialkylentriamin besteht,
bevorzugt ausgewählt aus der Gruppe bestehend aus 4,4'-Methylenbis(cyclohexylamin) und/oder Diethylentriamin unter Ausbildung von Copolymeren eingesetzt werden. Vorzugsweise kann das Amingemisch weiterhin Oxyalkylendiamine umfassen.

Als Oxyalkylendiamine eignen sich insbesondere solche Oxyalkylendiamine, die endständige Aminogruppen aufweisen. Ein bevorzugtes Oxyalkylendiamin ist eine Verbindung der allgemeinen Formel

NH₂[(CH₂)₂O)]ₙ(CH₂)₂NH₂

in welcher n eine ganze Zahl zwischen 2 und 6, bevorzugt zwischen 2 und 5, weiter bevorzugt zwischen 2 und 4 und insbesondere 2 ist. Bevorzugt sind Polyoxyethylendiamine, im Speziellen Triethylenglycoldiamin. Weiter bevorzugt eingesetzt werden können Polyoxypropylendiamine, insbesondere Di- oder Tripropylenglycoldiamin.

In einer bevorzugten Ausführungsform liegt der polymere oder oligomere Wirkstoff als Homopolymer vor. In diesen Fällen besteht das Amingemisch aus einem Diamin, das wenigstens einen cycloaliphatischen Rest aufweist, oder aus einem Dialkylentriamin.

In einer weiteren bevorzugten Ausführungsform besteht das Amingemisch aus dem Triamin Diethylentriamin. In dieser Variante liegt der polymere oder oligomere Wirkstoff somit als Homopolymer vor, beispielsweise als Poly(iminoimidazol).

In einer weiteren bevorzugten Ausführungsform besteht das Amingemisch aus dem Diamin 4,4'-Methylenbis(cyclohexylamin). Durch Polykondensation mit einem Guanidinsäureadditionssalz entsteht daraus beispielsweise das Homopolymer Poly(4,4'-methylenbis(cyclohexylaminhydrochlorid)).

Besonders bevorzugt sind die polymeren oder oligomeren Wirkstoffe, die erhältlich sind durch Polykondensation eines Guanidinsäureadditionssalzes mit einem Amingemisch, welches wenigstens ein Diamin, das wenigstens einen cycloaliphatischen Rest aufweist, umfasst. Diamine, die wenigstens einen cycloaliphatischen Rest aufweisen, sind beispielsweise cycloaliphatische Diamine, beispielsweise Cyclohexandiamin, Cyclopentandiamin sowie Derivate hiervon. Insbesondere bevorzugt sind solche Diamine, bei denen wenigstens eine NH₂-Gruppe direkt mit dem cycloaliphatischen Rest verknüpft ist. Besonders bevorzugt sind solche Diamine, bei denen beide NH₂-Gruppen jeweils direkt mit ein und denselben cycloaliphatischen Rest oder an verschiedenen cycloaliphatischen Resten verknüpft sind. In einer besonderen Ausführungsform umfasst das Amingemisch 4,4'-Methylenbis(cyclohexylamin).

In einer weiteren Ausführungsform der vorliegenden Erfindung umfasst das Amingemisch wenigstens ein Dialkylentriamin. Die Dialkylentriamine können dabei Alkylenreste unterschiedlicher Kettenlänge aufweisen. Bevorzugt sind allerdings Dialkylentriamine, bei denen die Alkylengruppen die gleiche Länge aufweisen. Bevorzugte Alkylenreste sind dabei Ethylen, Propylen und Butylen sowie Hexylen. In einer besonders bevorzugten Ausführungsform umfasst das Amingemisch das Triamin Diethylentriamin.

In einer weiteren bevorzugten Ausführungsform liegen die erfindungsgemäß verwendeten polymeren oder oligomeren Wirkstoffe in Form von Copolymeren vor. Diese können dabei sowohl willkürlich gemischt als auch als Blockcopolymere vorliegen. Im Falle von Copolymeren enthält das Amingemisch wenigstens zwei verschiedene Amine. Das Amingemisch enthält dabei eine erste Komponente und wenigstens eine zweite Komponente, wobei bevorzugt
a) die erste Komponente ein Diamin oder ein Triamin ist, das ausgewählt ist aus der Gruppe, die aus
   i) Diamin, das wenigstens einen cycloaliphatischen Rest aufweist, und
   ii) Dialkylentriamin besteht, und wobei
b) die zweite Komponente ein Diamin oder ein Triamin_ist, das ausgewählt ist aus der Gruppe, die aus
   i) Diamin, das wenigstens eine cycloaliphatischen Rest aufweist,
   ii) Dialkylentriamin,
   iii) Alkylendiamin und
   iv) Oxyalkylendiamin besteht, und
wobei die erste Komponente von der zweiten Komponente verschieden ist.

Als besonders geeignete copolymere oder cooligomere Wirkstoffe haben sich solche herausgestellt, bei denen die erste Komponente 4,4'-Methylenbis(cyclohexylamin) ist und die zweite Komponente ausgewählt ist aus Diethylentriamin, Hexamethylendiamin und Triethylenglycoldiamin.

In einer weiteren bevorzugten Ausführungsform enthält das copoylmere Guanidinderviat als erste Komponente Diethylentriamin und die zweite Komponente ist ausgewählt aus der Gruppe bestehend aus Hexamethylendiamin und Triethylenglycoldiamin.

Insbesondere bezüglich der bioziden Aktivität und des "Leaching"-Verhaltens bei der Einarbeitung der polymeren oder oligomeren Wirkstoffe in Kunststoffe, insbesondere thermoplastische Polymere, die dann zu medizinischen Artikeln verarbeitet werden, haben sich solche copolymeren Guanidinderivate als besonders geeignet herausgestellt, bei denen mindestens eine Komponente Alkylendiamin, insbesondere Hexamethylendiamin, ist. Im Speziellen bevorzugt sind polymere oder oligomere Wirkstoffe, die erhältlich sind durch Polykondensation eines Guanidinsäureadditionssalzes mit einem Amingemisch, bei dem die erste Komponente Diethylentriamin und/oder 4,4'-Methylenbis(cyclohexylamin) ist und die zweite Komponente Hexamethylendiamin.

Speziell bevorzugt sind Amingemische, die Diethylentriamin und Hexamethylendiamin umfassen. Die daraus erhältlichen Wirkstoffe zeigen nicht nur hervorragende antibakterielle Eigenschaften, sondern sind zusätzlich auch biokompatibel, was diese Wirkstoffe insbesondere für medizinische Artikel, die im direkten Kontakt zum Blutkreislauf stehen, eignet.

In einer weiteren Ausführungsform kann die erste Komponente Diamin, das wenigstens einen cycloaliphatischen Rest aufweist, und/oder ein Dialkylentriamin sein und die zweite Komponente ein Oxyalkylendiamin, insbesondere Triethylenglycoldiamin.

Bei der Herstellung von Copolymeren kann das Mischungsverhältnis der einzusetzenden Amine in weiten Bereichen variiert werden. Bevorzugt sind allerdings copolymere oder cooligomere Wirkstoffe, bei denen die Monomere des Amingemischs, also die erste Komponente und die zweite Komponente, in einem Molverhältnis von 4 : 1 bis 1 : 4, vorzugsweise 2 : 1 bis 1 : 2, vorliegt.

Die erfindungsgemäß einzusetzenden polymeren oder oligomeren Wirkstoffe besitzen bevorzugt ein mittleres Molekulargewicht (Gewichtsmittel) im Bereich von 500 bis 7000, insbesondere von 1000 bis 5000 Dalton.

Die erfindungsgemäß verwendeten polymeren oder oligomeren Wirkstoffe weisen allesamt eine antibakterielle Wirkung auf, die sich mit Hilfe der sogenannten "minimalen Hemmkonzentration" beschreiben lässt. Diese gibt die niedrigste Bakterizidkonzentration an, die das Wachstum von Bakterien in einer bestimmten Lösung hemmt. Besonders günstig ist dabei eine minimale Hemmkonzentration von weniger als 50 µg/ml. Bevorzugt weisen die erfindungsgemäß zu verwendenden polymeren polymeren oder oligomeren Wirkstoffe eine minimale Hemmkonzentration von weniger als 10 µg/ml, insbesondere von weniger als 5 µg/ml auf. Je geringer diese Konzentration ist, umso effektiver kann der entsprechende polymere oder oliogmere Wirkstoff als Biozid eingesetzt werden.

In einer bevorzugten Ausführungsform weisen die erfindungsgemäß einzusetzenden polymeren oder oligomeren Wirkstoffe eine minimale Hemmkonzentration von 50 µg/ml oder weniger, bevorzugt 30 µg/ml oder weniger, besonders bevorzugt 10 µg/ml oder weniger und insbesondere 5 µg/ml oder weniger auf.

Die erfindungsgemäß einzusetzenden polymeren oder oligomeren Wirkstoffe lassen sich relativ einfach herstellen. Die Polykondensation kann durch Mischen eines Äquivalents eines Säureadditionssalzes mit einem Äquivalent der Aminmischung und anschließendes Erhitzen, vorzugsweise in einem Bereich von 140 bis 180 °C und Rühren der Schmelze bei erhöhten Temperaturen, vorzugsweise in einem Bereich von 140 bis 180 °C, bis die Gasentwicklung beendet ist, erfolgen. Die Polykondensation erfolgt regelmäßig in einem Zeitraum von mehreren Stunden, bei der die Schmelze bevorzugt in dem Temperaturbereich von 140 bis 180 °C gerührt wird. Ein bevorzugter Reaktionszeitraum ist 1 bis 15 Stunden, vorzugsweise 5 bis 10 Stunden.

Je nach gewünschtem Endprodukt variiert das Verfahren leicht, so ist es zur Herstellung des Homopolymeren auf Basis von 4,4'-Methylenbis(cyclohexylamin) günstig, wenn die Reaktionstemperatur 170 °C beträgt. Das auf Basis von Diethylentriamin hergestellte Homopolymer kann dagegen bei 150 °C gewonnen werden. Die Herstellung der erfindungsgemäß einzusetzenden copolymeren Wirkstoffe erfolgt wiederum bevorzugt in einem Temperaturbereich von etwa 170 °C.

Es hat sich zudem überraschend gezeigt, dass beispielsweise bei der Kondensation von Triaminen cyclische Strukturen, beispielsweise Iminoimidazol-Strukturen, innerhalb der Polymereinheiten entstehen können, so dass es sich bei den erfindungsgemäß einzusetzenden Wirkstoffen nicht nur um polymere oder oligomere Guanidinderivate, sondern auch um polymere oder oligomere Iminoimidazolderivate handeln kann.

Insbesondere ist es vorteilhaft, wenn der polymere oder oligomere Wirkstoff eine Struktur aufweist, die aus der Gruppe umfassend 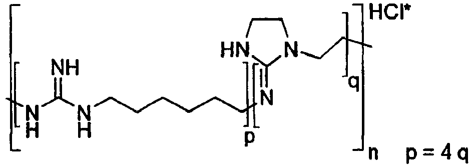 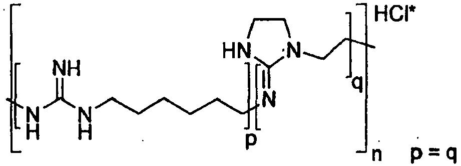 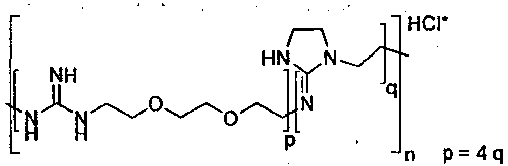 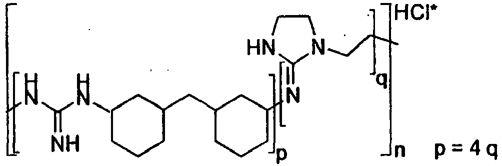 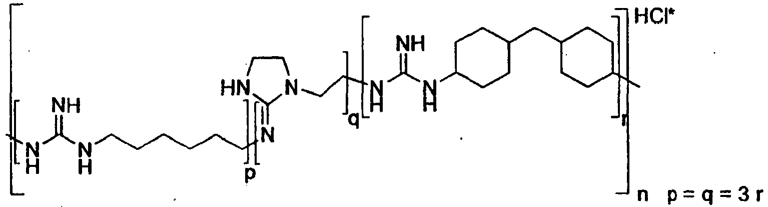 ausgewählt ist, wobei
HCl* bedeutet, dass das HCl nicht kovalent gebunden ist,
n ist eine natürliche Zahl, vorzugsweise von 1 bis 20, weiter bevorzugt von 2 bis 16 und insbesondere von 3 bis 8,
p, q und r sind ganze Zahlen, die das bevorzugte molare Verhältnis der Strukturfragmente zueinander in den Formeln definieren.

In einer bevorzugten Ausführungsform der vorliegenden Erfindung sind die polymeren oder oligomeren Wirkstoffe erhältlich durch Polykondensation eines Guanidinsäureadditionssalzes mit einem Amingemisch, welches wenigstens ein Diamin und/oder ein Triamin enthält, wobei wenigstens ein Amin ausgewählt ist aus der Gruppe, die aus
i) Diamin, das wenigstens einen cycloaliphatischen Rest aufweist, und
ii) Dialkylentriamin besteht,
und wobei die Mischung aus Guanidinsäureadditionssalz und Amingemisch auf eine Temperatur oberhalb von 140 °C, vorzugsweise in einem Bereich von 150 bis 170 °C, erwärmt wird und die Reaktionsmischung bei dieser Temperatur für wenigstens 1 Stunde, vorzugsweise wenigstens 5 Stunden, gehalten wird.

Erfindungsgemäß werden die polymeren oder oligomeren Wirkstoffe als Additive in Zusammensetzungen für medizinische Artikel eingesetzt. Je nach biozider Wirksamkeit der polymeren oder oligomeren Wirkstoffe sowie der Art und des Aufbaus des medizinischen Artikels können die Zusammensetzungen für den medizinischen Artikel in einer bevorzugten Ausführungsform den polymeren oder oligomeren Wirkstoff in einer Menge von maximal 10,0 Gew.-%, bevorzugt in einer Menge von 0,01 bis 5 Gew.-% und insbesondere in einer Menge von 1,0 bis 4,0 Gew.-%, jeweils bezogen auf die Zusammensetzung für den medizinischen Artikel, enthalten.

Ein besonderer Vorteil der erfindungsgemäß einzusetzenden polymeren oder oligomeren Wirkstoffe ist deren Einarbeitbarkeit in Kunststoffen, insbesondere thermoplastischen Polymeren, die häufig den wesentlichen Bestandteil von Zusammensetzungen für medizinische Artikel bilden. Es hat sich überraschend gezeigt, dass sich die polymeren oder oligomeren Wirkstoffe nicht nur problemlos in Kunstoffen, insbesondere thermoplastischen Polymerzusammensetzungen einarbeiten lassen, sondern dass darüber hinaus die mechanischen Eigenschaften, wie beispielsweise die Zugbelastbarkeit oder der Biegewiderstand, nur unwesentlich beeinflusst werden. Darüber hinaus zeigte sich überraschend, dass der Einsatz der polymeren oder oligomeren Wirkstoffe in Zusammensetzungen umfassend Kunststoffe, insbesondere thermoplastische Polymere, für medizinische Artikel bei der Verarbeitung zu extrem glatten Oberflächen führt und darüber hinaus einen steuerbaren "Leaching"-Effekt aufweist. So können die bioziden polymeren oder oligomeren Wirkstoffe so verarbeitet werden, dass sie nicht von Flüssigkeiten wie beispielsweise Wasser oder Ethanol aus dem Polymer-Blend freigesetzt werden, was beispielsweise für medizinische Artikel wie Katheter von Bedeutung ist. Andererseits kann eine gezielte Freisetzung auch wünschenswert sein, beispielsweise bei Wundauflagen. Die mit den polymeren oder oligomeren Wirkstoffen ausgerüsteten Zusammensetzungen, insbesondere Zusammensetzungen umfassend thermoplastische Polymere für medizinische Artikel, weisen eine hervorragende antimikrobielle Wirksamkeit auf, obschon sie wie in Falle der Katheteranwendungen keinen Leaching-Effekt aufweisen.

In einer bevorzugten Ausführungsform umfasst die Zusammensetzung für medizinische Artikel weiterhin Kunstoffe, vorzugsweise thermoplastische Polymere, insbesondere ausgewählt aus Polyurethan, Polyolefin, Polyvinylchlorid, Polycarbonat, Polystyrol, Polyethersulfon, Silikon und Polyamid. Besonders bevorzugt umfassen die Zusammensetzungen für medizinische Artikel Polyurethan, Polyethylen oder Polypropylen.

In einer besonders bevorzugten Ausführungsform kann der oligomere oder polymere Wirkstoff auch kovalent gebunden an dem thermoplastischen Polymer vorliegen. In einer Ausführungsform liegen in den Zusammensetzungen für die medizinischen Artikel mindestens 50 Gew.-%, vorzugsweise mindestens 75 Gew.-% und insbesondere mindestens 95 Gew.-% der eingesetzten oligomeren oder polymeren Wirkstoffe, kovalent gebunden an dem Thermoplast, vor.

Darüber hinaus können die Zusammensetzungen für medizinische Artikel weitere übliche Zusatzstoffe enthalten. Hier kommen insbesondere unter physiologischen Bedingungen inerte Füllstoffe in Frage. Besonders geeignet ist Bariumsulfat. Beispielsweise kann ein geeignetes BaSO₄ unter dem Handelsnamen Blancfix^{®} von der Firma Sachtleben Chemie GmbH bezogen werden. Die Füllstoffe liegen bevorzugt in den Zusammensetzungen für medizinische Artikel in einer Menge von 10 bis 35 Gew.-%, bezogen auf die Gesamtmischung, vor. Vorteilhafterweise weisen die Füllstoffe eine mittlere Teilchengröße von 0,01 µm bis 10 µm auf.

In einer bevorzugten Ausgestaltung der vorliegenden Erfindung ist die Zusammensetzung jedoch im Wesentlichen frei von silikatischen Füllstoffen, da diese die Oberflächenglätte als auch den Leachingeffekt negativ beeinflussen können.

Im Wesentlichen frei im Rahmen der vorliegenden Erfindung bedeutet, dass die silikatischen Füllstoffe in einer Menge unterhalb von 1 Gew.-%, bevorzugt unterhalb von 0,5 Gew.-%, weiter bevorzugt unterhalb von 0,01 Gew.-% und insbesondere frei von jeglichen silikatischen Füllstoffen, vorliegen können, wobei sich die Gewichtsangaben auf das Gesamtgewicht der Zusammensetzung für die Herstellung des medizinischen Artikels bezieht.

Medizinische Artikel im Sinne der vorliegenden Erfindung sind insbesondere ausgewählt aus der Gruppe bestehend aus zentralnervösen Kathetern; peripheren Venenkathetern; Beatmungsschläuche; Stents; Produkten für Anwendung in der Regionalanästhesie, insbesondere Katheter, Kuppelungen, Filter; Produkten für die Infusionstherapie, insbesondere Behältnisse, Ports, Überleitungssysteme, Filter; Zubehör wie Konnektoren, Spikes, Ventile, Dreiwegehähne, Spritzen, Leitungen, Zuspritzports; Produkte der Zubereitung, insbesondere Transfersets, Mixsets; urologische Produkte, insbesondere Katheter, Urinmess- und -sammelgeräte; Wunddrainage; Wundversorgung; chirurgische Nahtmaterialien; Implantationshilfsstoffe sowie Implantate, insbesondere Kunststoffimplantate, beispielsweise Herniennetze, Vliese, Gestricke, Gewirke, Ports, Portkatheter, Gefäßprothesen; Desinfektionsmittel; chirurgisches Einmalinstrumentarium; Thoraxdrainagen; Sonden; Katheter; Gehäuse von medizinischen Geräten, insbesondere Infusionspumpen, Dialysegeräte und Monitore; künstliche Gebisse; Behälter für Flüssigkeiten, insbesondere Kontaktlinsenbehälter.

Medizinische Artikel im Rahmen der vorliegenden Erfindung erfassen auch Zubehörteile für medizinische Produkte, wie beispielsweise Spritzgussteile und Formteile. Eine besondere Bedeutung kommt der Verwendung der polymeren oder oligomeren Wirkstoffe als Additiv in Beschichtungen für chirurgisches Nahtmaterial zu.

Ein bevorzugter medizinischer Artikel im Rahmen der vorliegenden Erfindung ist eine Wundauflage.

Besonders bevorzugte medizinische Artikel sind schlauchförmige medizinische Artikel. Solche Artikel weisen zumindest einen schlauchförmigen Bestandteil auf.

Schlauchförmige medizinische Artikel im Sinne der vorliegenden Erfindung sind solche medizinischen Artikel, die Fluide führen können. Insbesondere sind die medizinischen Artikel ausgewählt aus der Gruppe bestehend aus Katheder; zentralvenösen Kathetern; peripheren Venenkathetern; Beatmungsschläuche; Stents; Kuppelungen; Ports; Überleitungssysteme; Konnektoren, Spikes, Ventile, Dreiwegehähne, Spritzen, Leitungen, Zuspritzports; Wunddrainage; Thoraxdrainagen und Sonden.

Besonders bevorzugte medizinische Artikel sind Katheter, insbesondere solche, die durch Extrusion von Zusammensetzungen umfassend Polyurethan und/oder Polyethylen und/oder Polyamid, hergestellt werden.

Ein besonders geeignetes Polyamid ist unter dem Handelsnamen Pebax^{®} (Arkema) erhältlich. Es handelt sich dabei um ein Polyamid, das Polyether-Blöcke aufweist.

Aufgrund ihrer hervorragenden antimikrobiellen Wirkung sind die polymeren oder oligomeren Wirkstoffe auch als Additiv für Reinigungsmittel oder Desinfektionsmittel, insbesondere Händedesinfektionsmittel geeignet.

Ein weiterer Gegenstand der vorliegenden Erfindung ist ein Verfahren zur Herstellung eines medizinischen Artikels, vorzugsweise eines schlauchförmigen medizinischen Artikels, umfassend die folgenden Schritte:
a) Zusammenführen und Vermischen eines polymeren oder oligomeren Wirkstoffs mit biozider Wirkung, welcher erhältlich ist durch Polykondensation eines Guanidin-Säureadditionssalzes mit einem Amingemisch, welches wenigstens ein Diamin und/oder ein Triamin enthält, wobei wenigstens ein Amin ausgewählt ist aus der Gruppe, die aus
   i) Diamin, welches mindestens einen cycloaliphatischen Rest aufweist, und
   ii) Dialkylentriamin
   besteht, mit mindestens einem Kunststoff, insbesondere einem thermoplastischen Polymer,
b) Einsetzen des unter a) erhaltenen Gemisches in einem oder mehreren Formgebungsverfahren unter Ausbildung eines medizinischen Artikels, vorzugsweise eines schlauchförmigen medizinischen Artikels.

Bevorzugte polymere oder oligomere Wirkstoffe und bevorzugte Kunststoffe entsprechen den zuvor genannten.

Das Vermischen in Schritt a) erfolgt bevorzugt durch Schmelzkneten. Das polymere Guanidinderivat kann als wässrige Lösung dem geschmolzenen Kunststoff zugegeben werden und anschließend in einem Extruder vermischt werden. Bevorzugt erfolgt das Schmelzkneten bei Temperaturen oberhalb von 100°C, weiter bevorzugt oberhalb von 150°C.

Bevorzugt wird der polymere oder oligomere Wirkstoff dem Formgebungsverfahren in Schritt b) als Granulat oder Masterbatch zugeführt. Die Herstellung von Granulaten kann nach dem Fachmann auf dem Gebiet der Kunststofftechnologie geläufigen Verfahren erfolgen. Bevorzugt ist der Masterbatch ein Granulat, das den polymeren oder oligomeren Wirkstoff in einer höheren Konzentration als der vorgesehenen Endkonzentration im medizinischen Artikel aufweist. Bei Einsatz eines Masterbatches wird dieser daher durch Zugabe von weiterem Kunststoff weiter auf die gewünschte Endkonzentration verdünnt.

Das in Schritt b) des erfindungsgemäßen Verfahrens zum Einsatz kommende Formgebungsverfahren ist bevorzugt ein Extrusionsverfahren. Hiermit können beispielsweise schlauchartige Komponenten des Katheters hergestellt werden.

Die Formgebungsverfahren werden vorzugsweise bei Temperaturen oberhalb des Schmelzpunkts des in Schritt a) hergestellten Gemisches durchgeführt, besonders bevorzugt in einem Temperaturbereich oberhalb von 160 °C, weiter bevorzugt in einem Bereich von 180 - 260 °C.

Als Ergebnis entsteht ein Werkstoff mit antimikrobiellen Eigenschaften bei welchem das Additiv (polymeres oder oligomerer Wirkstoff) physikalisch eingemischt oder ggf. chemisch an den jeweiligen Kunststoff gebunden ist.

Ein weiterer Gegenstand der vorliegenden Erfindung ist ein medizinischer Artikel umfassend einen polymeren oder oligomeren Wirkstoff mit biozider Wirkung, welches erhältlich ist durch Polykondensation eines Guanidin-Säureadditionssalzes mit einem Amingemisch, welches wenigstens ein Diamin und/oder ein Triamin enthält, wobei wenigstens ein Amin ausgewählt ist aus der Gruppe, die aus
i) Diamin, das wenigstens einen cycloaliphatischen Rest aufweist, und
ii) Dialkylentriamin besteht,
sowie mindestens einen Kunststoff, insbesondere ein thermoplastisches Polymer. Bevorzugte polymere oder oligomere Wirkstoffe und bevorzugte Kunststoffe entsprechen den zuvor genannten.

Das in dem erfindungsgemäßen medizinischen Artikel sowie in dem erfindungsgemäßen Verfahren einzusetzende thermoplastische Polymer ist bevorzugt ein thermoplastisches Polyurethan. Es haben sich hier als besonders geeignet Polyurethane herausgestellt, die aus einer Kombination von 4,4'-Diphenylmethan-diisocyanat (MDI) und einem Polyol auf Polyester- oder Polyetherbasis bestehen. Vorteilhafterweise umfasst das Polyol einen Polytetramethylenglycolether. Weitere geeignete thermoplastische Polymere, die bevorzugt für Zusammensetzungen der erfindungsgemäßen medizinischen Artikel in Frage kommen, sind beispielsweise ausgewählt aus Polyurethan, Polyolefin, Polyvinylchlorid, Polycarbonat, Polystyrol, Polyethersulfon, Silikon und Polyamid. Besonders bevorzugt umfassen die Zusammensetzungen für medizinische Artikel Polyurethan, Polyethylen oder Polypropylen oder Polyamid.

Bevorzugte medizinische Artikel entsprechen den zuvor genannten. Insbesondere bevorzugte medizinische Artikel sind Wundauflagen und Katheter.

Weitere Merkmale, Einzelheiten und Vorteile der Erfindung ergeben sich aus dem Wortlaut der Ansprüche sowie aus der folgenden Beschreibung von Ausführungsbeispielen.

### Beispiel 1 - Synthese von Poly(4,4'-methylenbis(cyclohexylamin)-guanidinhydrochlorid) (PMBCG)

In einen dreifach ausgeheizten 100 ml-Dreihalskolben wird im Argon-Gegenstrom 1 Äquivalent (8,12 g, 85 mmol) Guanidinhydrochlorid gegeben. Anschließend wird in der Glovebox 1 Äquivalent (17,88 g, 85 mmol) 4,4'-Methylenbis(cyclohexylamin)zugefügt.

Der Kolben wird mit einem Innenthermometer und einem dreifach ausgeheizten Rückflusskühler mit Rückschlagventil nach Stutz (im Folgenden Stutzkühler) ausgestattet.

In einem Ölbad wird das Reaktionsgemisch erwärmt, wobei ab einer Temperatur von 100°C eine langsame Gasentwicklung einsetzt. Bei weiterer Temperaturerhöhung wird die Gasentwicklung nur langsam stärker. Nach insgesamt 85 Minuten wird eine Temperatur von 170°C erreicht.

Diese Temperatur wird für neun Stunden beibehalten, bis die Gasentwicklung nach Augenschein beendet ist.

Unter Eiskühlung und Ölpumpenvakuum wird die Schmelze auf Raumtemperatur abgekühlt.

Die eingesetzten Ausgangsmengen liefern unter den zuvor genannten Bedingungen 24,48 g eines transparenten, farblosen und spröden Feststoffs.

Die Struktur des erhaltenen Polymers lässt sich entsprechend Formel (I) darstellen.

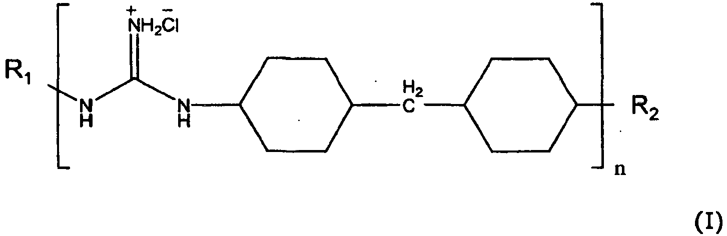

Dabei ist n = 1 bis 8, vorwiegend 1 bis 3.

Die Reste R1 und R2 können sowohl von dem eingesetzten Monomer als auch von dem eingesetzten Guanidinhydrochlorid stammen und sind daher wie folgt definiert:
R1 ist ausgewählt aus H oder 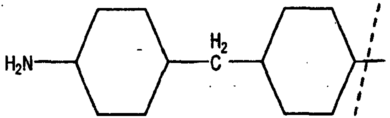 und
R2 ist ausgewählt aus NH₂ oder

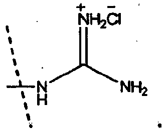

Das entstehende Produktgemisch enthält somit polymere Verbindungen entsprechend den Formeln (II), (III) und (IV): 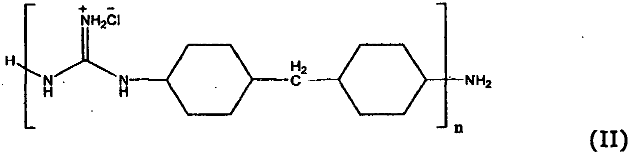 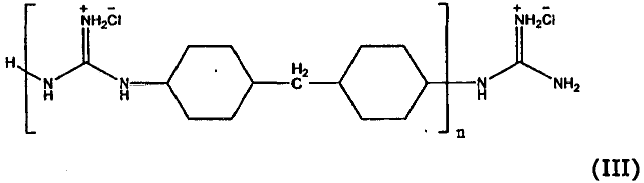 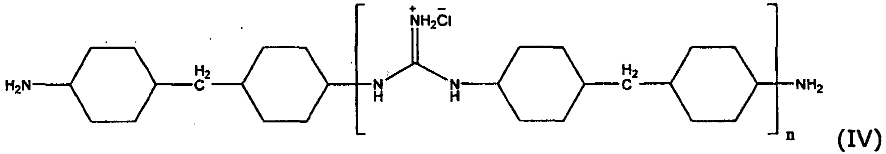 wobei n definiert ist wie in Formel (I).

### Beispiel 2 - Synthese eines Homopolymers auf Basis von Diethylentriamin

In einem dreifach ausgeheizten und mit Argon befüllten 100 ml-Dreihalskolben mit Innenthermometer, Stutzkühler und einem Absaugstück mit Hahn werden 1 Äquivalent (8,12 g, 85 mmol) Guanidinhydrochlorid und 1 Äquivalent (8,77 g, 85 mmol) Diethylentriamin mit Hilfe eines Ölbades innerhalb von 50 Minuten auf eine Temperatur von 150°C erwärmt.

Ab dem Erreichen einer Temperatur von 95°C ist eine Gasentwicklung zu beobachten, die bei weiterer Erhöhung der Temperatur schnell zunimmt.

Die Schmelze wird unter Rühren für fünf Stunden bei 150°C gehalten bis die Gasentwicklung beendet ist.

Unter Eiskühlung und Ölvakuum wird die Schmelze auf Raumtemperatur abgekühlt.

Die eingesetzten Ausgangsmengen liefern unter den zuvor genannten Bedingungen 11,96 g eines weißen und spröden Feststoffs.

Überraschenderweise zeigt die repetitive Monomereinheit des entstehenden polymeren Wirkstoffs die zyklische Struktur entsprechend Formel (V):

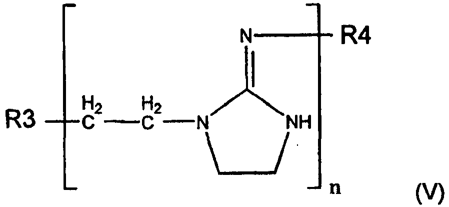

Dabei ist n = 1 bis 12, vorwiegend 2 bis 8.

R3 ist entweder NH₂ oder 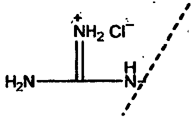 und R4 ist ausgewählt aus

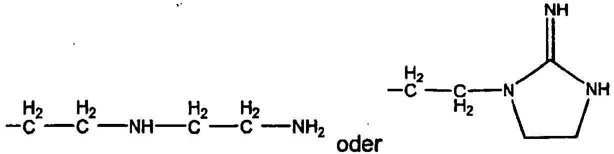

Das entstehende Produktgemisch enthält somit polymere Verbindungen entsprechend den Formeln (VI), (VII) und (VIII):

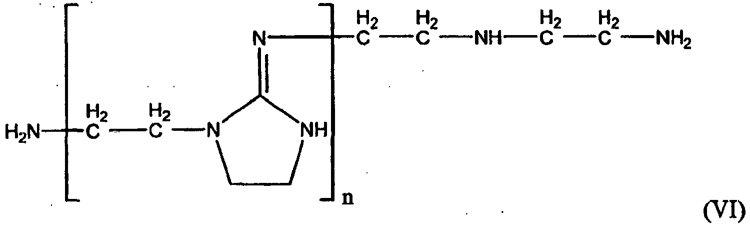

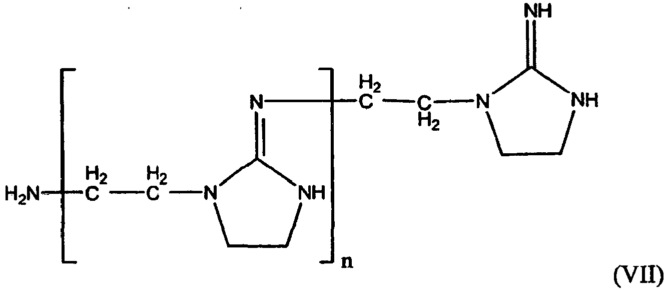

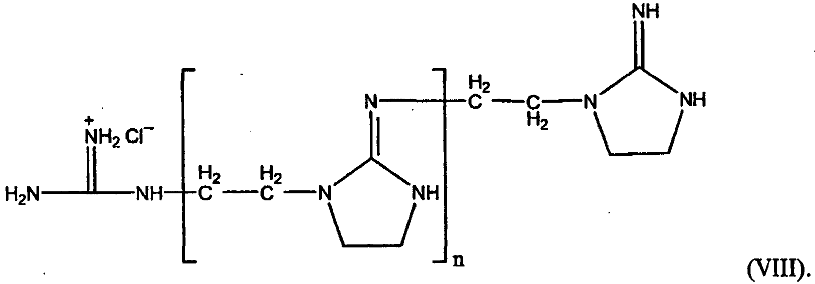

Dabei ist vorstellbar, dass etwa 90 % der Ringe in den Formeln (VI), (VII) und (VIII) eine positive Ladung tragen. Denkbar ist dabei auch, dass die positive Ladung nicht auf einem der Stickstoffatome im Ring lokalisiert sondern vielmehr delokalisiert ist. Eine alternative Darstellungsform der Formel (VIII) ist mithin die folgende Formel (VIII')

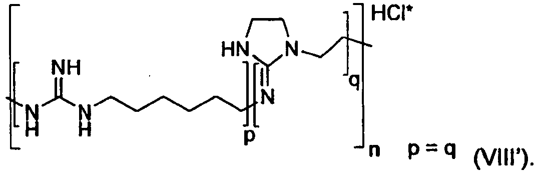

Auch die Formeln (I) bis (IV) des Beispiels 2 lassen sich in analoger Weise darstellen, wobei die positive Ladung mesomer auf alle drei Stickstoffatome der Guanidineinheit verteilt ist.

### Beispiel 3 - Synthese von Guanidin-Copolymeren

In einem entsprechend den vorbeschriebenen Beispielen vorbereiteten Reaktionskolben werden jeweils 1 Äquivalent (8,12 g, 85 mmol) Guanidinhydrochlorid und 1 Äquivalent der Comonomere, die in einem Mischungsverhältnis entsprechend Tabelle 1 vorliegen, gemeinsam mit Hilfe eines Ölbades innerhalb von 30 Minuten auf eine Temperatur von 170°C erwärmt.

Die Schmelze wird unter Rühren für fünf Stunden bei dieser Temperatur gehalten. Unter Eiskühlung und Ölvakuum wird die Schmelze auf Raumtemperatur abgekühlt.

**Tabelle 1: Mischungsverhältnisse der in dem Amin-Gemisch eingesetzten Di- und Triamine zur Herstellung von Guanidin-Copolymeren (Äq = Äquivalent).**

| Nr. | Monomer 1 | Monomer 2 | Eingesetzte Menge Monomer 1 | Eingesetzte Menge Monomer 2 |
|---|---|---|---|---|
| C1 | 4,4'-Methylenbis(cyclohexyl-amin) | Diethylentriamin | 14,30 g | 2,21 g |
| | | | 68 mmol | 17 mmol |
| | | | 0,80 Äq | 0,2 Äq |
| C2 | 4,4'-Methylenbis(cyclohexyl-amin) | Diethylentriamin | 13,41 g | 2,77 g |
| | | | 63,75 mmol | 21,25 mmol |
| | | | 0,75 Äq | 0,25 Äq |
| C3 | 4,4'-Methylenbis(cyclohexyl-amin) | Diethylentriamin | 11,92 g | 3,69 g |
| | | | 56,67 mmol | 28,33 mmol |
| | | | 0,67 Äq | 0,33 Äq |
| C4 | 4,4'-Methylenbis(cyclohexyl-amin) | Diethylentriamin | 8,94 g | 5,53 g |
| | | | 42,50 mmol | 42,50 mmol |
| | | | 0,50 Äq | 0,50 Äq |
| C5 | 4,4'-Methylenbis(cyclohexyl-amin) | Hexamethylen-diamin | 3,58 g | 7,90 g |
| | | | 17,00 mmol | 68,00 mmol |
| | | | 0,20 Äq | 0,80 Äq |
| C6 | 4,4'-Methylenbis(cyclohexyl-amin) | Hexamethylen-diamin | 4,47 g | 7,41 g |
| | | | 21,25 mmol | 63,75 mmol |
| | | | 0,25 Äq | 0,75 Äq |
| C7 | 4,4'-Methylenbis(cyclohexyl-amin) | Hexamethylen-diamin | 5,96 g | 6,59 g |
| | | | 28,33 mmol | 56,67 mmol |
| | | | 0,33 Äq | 0,67 Äq |
| C8 | 4,4'-Methylenbis(cyclohexyl-amin) | Hexamethylen-diamin | 8,94 g | 4,94 g |
| | | | 42,50 mmol | 42,50 mmol |
| | | | 0,50 Äq | 0,50 Äq |
| C9 | 4,4'-Methylenbis(cyclohexyl-amin) | Hexamethylen-diamin | 11,92 g | 3,29 g |
| | | | 56,67 mmol | 28,33 mmol |
| | | | 0,67 Äq | 0,33 Äq |
| C10 | 4,4'-Methylenbis(cyclohexylam in) | Hexamethylen-diamin | 13,41 g | 2,47 g |
| | | | 63,75 mmol | 21,25 mmol |
| | | | 0,75 Äq | 0,25 Äq |
| C11 | 4,4'-Methylenbis(cyclohexyl-amin) | Hexamethylen-diamin | 14,30 g | 1,98 g |
| | | | 68,00 mmol | 17,00 mmol |
| | | | 0,80 Äq | 0,20 Äq |
| C12 | 4,4'-Methylenbis(cyclohexylam in) | Triethylenglycol-diamin | 13,41 g | 3,15 g |
| | | | 63,75 mmol | 21,25 mmol |
| | | | 0,75 Äq | 0,25 Äq |
| C13 | 4,4'-Methylenbis(cyclohexyl-amin) | Triethylenglycol-diamin | 11,92 g | 4,20 g |
| | | | 56,67 mmol | 28,33 mmol |
| | | | 0,67 Äq | 0,33 Äq |
| C14 | 4,4'-Methylenbis(cyclohexyl-amin) | Triethylenglycol-diamin | 8,94 g | 6,30 g |
| | | | 42,50 mmol | 42,50 mmol |
| | | | 0,50 Äq | 0,50 Äq |
| C15 | 4,4'-Methylenbis(cyclohexylam in) | Triethylenglycol-diamin | 5,96 g | 8,40 g |
| | | | 28,33 mmol | 56,67 mmol |
| | | | 0,33 Äq | 0,67 Äq |
| C16 | 4,4'-Methylenbis(cyclohexyl-amin) | Triethylenglycol-diamin | 4,47 g | 9,45 g |
| | | | 21,25 mmol | 63,75 mmol |
| | | | 0,25 Äq | 0,75 Äq |
| C17 | Diethylentriamin | Hexamethylen-diamin | 1,75 g | 7,90 g |
| | | | 17,00 mmol | 68,00 mmol |
| | | | 0,20 Äq | 0,80 Äq |
| C18 | Diethylentriamin | Hexamethylen-diamin | 2,19 g | 7,41 g |
| | | | 21,25 mmol | 63,75 mmol |
| | | | 0,25 Äq | 0,75 Äq |
| C19 | Diethylentriamin | Hexamethylen-diamin | 3,69 g | 6,59 g |
| | | | 28,33 mmol | 56,67 mmol |
| | | | 0,33 Äq | 0,67 Äq |
| C20 | Diethylentriamin | Hexamethylen-diamin | 5,53 g | 4,94 g |
| | | | 42,50 mmol | 42,50 mmol |
| | | | 0,50 Äq | 0,50 Äq |
| C21 | Diethylentriamin | Triethylenglycoldi amin | 8,30 g | 3,15 g |
| | | | 63,75 mmol | 21,35 mmol |
| | | | 0,75 Äq | 0,25 Äq |
| C22 | Diethylentriamin | Triethylenglycol-diamin | 7,38 g | 4,20 g |
| | | | 56,67 mmol | 28,33 mmol |
| | | | 0,67 Äq | 0,33 Äq |
| C23 | Diethylentriamin | Triethylenglycoldi amin | 5,53 g | 6,30 g |
| | | | 42,50 mmol | 42,50 mmol |
| | | | 0,50 Äq | 0,50 Äq |

### Beispiel 4 - Bestimmung der minimalen Hemmkonzentration der erfindungsgemäß verwendeten polymeren Guanidinderivate.

Zur Überprüfung der bioziden Wirkung der erfindungsgemäß verwendeten polymeren Guanidinderivate werden die Verbindungen, die entsprechend einem der vorangegangenen Beispiele hergestellt werden in einem Bakterien-Nährmedium, vorzugsweise Tryptic Soy Broth, angesetzt und auf verschiedene Konzentrationen verdünnt.

Diese Lösungen unterschiedlicher Konzentration werden mit einer Suspension von *Escherichia coli* inokuliert und für 24 h bei 37°C inkubiert.

Unter der minimalen Hemmkonzentration (MHK) wird dann die geringste Konzentration des zu untersuchenden Biozids in der Lösung verstanden, bei der das Wachstum der Bakterien gehemmt wird. Bei der entsprechenden Lösung ist dann keine Eintrübung durch das Wachstum der Bakterien beobachtbar.

Für die in Beispiel 1 und Beispiel 2 dargestellten Homopolymere entsprechend Formel (I) und Formel (V) sowie für die aus den in Beispiel 3 genannten Comonomer-Mischungen C1 bis C23 erhaltenen Copolymere werden die in Tabelle 2 aufgeführten mittleren minimalen Hemmkonzentrationen (MHK) erhalten.

**Tabelle 2: Bestimmung der minimalen Hemmkonzentration erfindungsgemäß verwendeter polymerer Guanidinderivate (MHK = minimale Hemmkonzentration).**

| **Verbindung** | **MHK [µg/ml]** | **Verbindung** | **MHK [µg/ml]** |
|---|---|---|---|
| Kontrollpolymer | 5 | C11 | 9,75 |
| Entspr. Formel (I) | 5 | C12 | 5,5 |
| Entspr. Formel (V) | >250 | C13 | 8,5 |
| C1 | 7,5 | C14 | 10 |
| C2 | 22,5 | C15 | 10 |
| C3 | 25 | C16 | 10 |
| C4 | 50 | C17 | 3 |
| C5 | 1,5 | C18 | 10 |
| C6 | 4,7 | C19 | 10 |
| C7 | 4,25 | C20 | 40 |
| C8 | 2,5 | C21 | >50 |
| C9 | 3,5 | C22 | >50 |
| C10 | 2,5 | C23 | >50 |

Als Kontrolle wurde ein Kontrollpolymer eingesetzt, dessen biozide Wirkung bekannt ist und dessen minimale Hemmkonzentration üblicherweise bei 5 µg/ml liegt.

Man erkennt, dass alle erfindungsgemäß verwendeten polymeren Guanidinderivate, insbesondere die erfindungsgemäß verwendeten Copolymere eine biozide Wirkung aufweisen. Dabei weisen insbesondere Copolymere, die als zweites Monomer Hexametyhlendiamin aufweisen, eine minimale Hemmkonzentration auf, die sogar unterhalb von 5 µg/ml liegt:

**Tabelle 3: Ausgewählte erfindungsgemäß verwendete Copolymere mit besonders niedriger minimaler Hemmkonzentration (MHK). (MBC = 4,4'-Methylenbis(cyclohexylamin), HMD = Hexamethylendiamin, DETA = Diethylentriamin).**

| **Copolymer** | **Monomer 1** | **Monomer 2** | **Mischungs-verhältnis** | **Reaktions-Bedingungen** | **MHK** |
|---|---|---|---|---|---|
| C5 | MBC | HMD | 1:4 | 5h, 170°C | 1,5 |
| C6 | MBC | HMD | 1:3 | 5h, 170°C | 4,7 |
| C7 | MBC | HMD | 1:2 | 5h, 170°C | 4,25 |
| C8 | MBC | HMD | 1:1 | 5h, 170°C | 2,5 |
| C9 | MBC | HMD | 2:1 | 5h, 170°C | 3,5 |
| C10 | MBC | HMD | 3:1 | 5h, 170°C | 2,5 |
| C17 | DETA | HMD | 1:4 | 5h, 170°C | 3 |

Man erkennt, dass es bei den erfindungsgemäß verwendeten polymeren oder oligomeren Wirkstoffen mit biozider Wirkung, wobei der Wirkstoff das Produkt einer Polykondensation eines Guanidin-Säureadditionssalzes mit einem Amin-Gemisch, welches wenigstens ein Diamin und/oder ein Triamin enthält, besonders günstig ist, wenn wenigstens ein Amin ausgewählt ist aus 4,4'-Methylenbis(cyclohexylamin) und Diethylentriamin. Zweckmäßig ist dabei das Guanidin-Säureadditionssalz Guanidinhydrochlorid.

Man erkennt weiter, dass das polymere oder oligomere Wirkstoff ein Homopolymer sein kann. Dabei ist es günstig, wenn das Amin-Gemisch aus dem Triamin Diethylentriamin besteht oder wenn das Amin-Gemisch aus dem Diamin 4,4'-Methylenbis(cyclohexylamin) besteht.

Man erkennt auch, dass das Amin-Gemisch eine erste Komponente und wenigstens eine zweite Komponente enthalten kann, wobei die erste Komponente ein Diamin oder ein Triamin ist, ausgewählt aus der Gruppe 4,4'-Methylenbis(cylcohexylamin), Diethylentriamin, und wobei die zweite Komponente ein Diamin oder ein Triamin, ausgewählt aus der Gruppe 4,4'-Methylenbis(cylcohexylamin), Diethylentriamin, Hexamethylen-diamin, Triethylenglycol-diamin, ist und wobei die erste Komponente von der zweiten Komponente verschieden ist.

Besonders bevorzugt ist die erste Komponente 4,4'-Methylenbis(cylcohexylamin) und die zweite Komponente ist ausgewählt aus Diethylentriamin, Hexamethylendiamin, Triethylenglycoldiamin. Günstig ist es auch, wenn die erste Komponente Diethylentriamin ist und die zweite Komponente ausgewählt ist aus Hexamethylendiamin und Triethylenglycoldiamin.

Dabei liegen die erste Komponente und die zweite Komponente bevorzugt in einem Mischungsverhältnis von 4:1 bis 1:4 vor. Das Amin-Gemisch und das Guanidin-Salz werden bevorzugt in etwa äquimolar zueinander eingesetzt.

Man erkennt, dass es bei den erfindungsgemäß verwendeten polymeren oder oligomeren Wirkstoffen, wobei der Wirkstoff das Produkt einer Polykondensation eines Guanidin-Säureadditionssalzes mit einem Amin-Gemisch, welches wenigstens ein Diamin und/oder ein Triamin enthält, besonders vorteilhaft ist, wenn sie nach einem Verfahren hergestellt sind, umfassend die Schritte Vorlegen von etwa einem Äquivalent Guanidinhydrochlorid, Zugeben etwa eines Äquivalents einer Amin-Mischung, welche eine oder zwei der Verbindungen der Gruppe umfassend Diamin, das wenigstens einen cycloaliphatischen Rest aufweist, und Dialkylentriamin, enthält, Erhitzen auf 150 bis 170°C und Rühren der Schmelze bei 150 bis 170°C bis die Gasentwicklung beendet ist, wenigstens aber für 5 Stunden.

### Beispiel 5 - Allgemeine Vorschrift zur Fertigung eines Katheterschlauchs auf Polyurethan-Basis aus mit dem erfindungsgemäß einzusetzenden polymeren oder oligomeren Wirkstoff ausgestatteten TPU Granulat

Es wird ein aliphatisches thermoplastisches Polyurethan (auf Basis eines Polytetramethylen-Glycolethers) mit 10 bis 35 Gew.-%, bezogen auf die Gesamtmischung, Bariumsulfat mit einer mittleren Teilchengröße von 0,01 µm bis 10 µm sowie 0,5 bis 10 Gew.-% eines erfindungsgemäß zu verwendenden polymeren oder oligomeren Wirkstoffs gemischt und anschließend extrudiert. Die Extrusion erfolgt mit für die Katheterherstellung üblichen Extrudern, beispielsweise dem Extruder Maillefer Typ ED 45-30D.

### Beispiel 6 - Herstellung eines Katheterschlauchs

Es wird das thermoplastische Polyurethan Pellethane^{®} 2363-90 A (Lubrizol Advanced Materials; USA) mit 25 Gew.-% Bariumsulfat mit einer mittleren Korngröße von 0,7 µm und 3 Gew.-% des Wirkstoffs C20 gemischt und anschließend extrudiert. Die Extrusion erfolgte mit dem Extruder Maillefer Typ ED 4530D bei Temperaturen oberhalb von 160°C.

### Beispiel 7 (Vergleichsbeispiel)

Es wird ein Katheterstück entsprechend Beispiel 6 hergestellt, allerdings ohne Zusatz eines Polyguanidins. Die gemäß Beispiel 6 und 7 hergestellten Katheterschlauchstücke wurden durch rasterelektronenmikroskopische Aufnahmen untersucht. Es konnte rein optisch bereits festgestellt werden, dass die erfindungsgemäßen Katheterschlauchstücke wesentlich glatter ausgeformt waren als die Katheterschlauchstücke gemäß dem Vergleichsbeispiel 7.

Es wurde zusätzlich die Oberflächenrauhigkeit R_{z} [µm] gemessen. Für das Vergleichsbeispiel 7 wurde eine Oberflächenrauhigkeit R_{z} von 2,91 µm und für das erfindungsgemäße Katheterschlauchstück gemäß Beispiel 6, wurde lediglich eine Oberflächenrauhigkeit R_{z} von 2,32 µm festgestellt.

### Beispiel 8 (Vergleichsbeispiel)

Es wird ein Katheterschlauchstück entsprechend Beispiel 6 hergestellt, allerdings wird anstelle des erfindungsgemäß einzusetzenden Polyguanidins das Poly[2-(2-ethoxy-ethoxyethyl)-guanidiniumhydrochlorid] in einer Menge von 3 Gew.-% eingesetzt.

Die Katheterschlauchstücke gemäß Beispiel 6 (erfindungsgemäß) und gemäß Vergleichsbeispiel 8, wurden einem Proliferationstest unterzogen.

Der Proliferationstest basiert auf der Veröffentlichung Nature Medicine, Vol. 6, No. 8, 1053-1056; 2000. Hierzu werden die zu untersuchenden Katheterschlauchstücke mit unterschiedlichen Keimen kontaminiert und anschließend das Keimwachstum im Vergleich zu einer Probe, die nicht antimikrobiell ausgerüstet ist, beobachtet. Gemessen wird die Zeit, die das Keimwachstum benötigt, um einen vorgegebenen Wert (0,2 onset OD) im Vergleich zu einer nicht antimikrobiell ausgerüsteten Probe zu gelangen. Desto länger die Zeit, desto höher ist die antimikrobielle Wirksamkeit der Probe gegen die einzelnen Keime.

Tabelle 4 zeigt die Ergebnisse des Proliferationstests anhand unterschiedlicher Keime.

**Tabelle 4**

| Keim | Beispiel 8 [h] | Beispiel 6 [h] |
|---|---|---|
| MRSA | 21,0 | 48,0 |
| Staphylococcus epidermidis | 35,5 | 48,0 |
| Staphylococcus aureus | 26,8 | 48,0 |
| Pseudomonas aeruginosa | 0,1 | 48,0 |
| Enterococcus faecalis | 0,3 | 48,0 |
| Klebsielle pneumoniae | | 48,0 |

Anhand der Ergebnisse in Tabelle 4 wird deutlich, dass die erfindungsgemäß ausgerüsteten Katheterschlauchstücke signifikant wirksamer gegen MRSA, Staphylococcus epidermidis, Staphylococcus aureus, Pseudomonas aeruginosa und Enterococcus faecalis sind.

## Patentansprüche

1. Verwendung eines polymeren oder oligomeren Wirkstoffs mit biozider Wirkung, welcher erhältlich ist durch Polykondensation eines Guanidin-Säureadditionssalzes mit einem Amingemisch, welches wenigstens ein Diamin und/oder ein Triamin enthält, wobei wenigstens ein Amin ausgewählt ist aus der Gruppe, die aus
i) Diamin, das wenigstens einen cycloaliphatischen Rest aufweist, und
ii) Dialkylentriamin besteht,
als Additiv in einer Zusammensetzung für medizinische Artikel.

2. Verwendung gemäß Anspruch 1, **dadurch gekennzeichnet, dass** das Amingemisch das Diamin 4,4'-Methylenbis(cyclohexylamin) umfasst.

3. Verwendung gemäß mindestens einem der Ansprüche 1 oder 2, **dadurch gekennzeichnet, dass** das Amingemisch eine erste Komponente und wenigstens eine zweite Komponente enthält, wobei
a) die erste Komponente ein Diamin oder ein Triamin ist, das ausgewählt ist aus der Gruppe, die aus
i) Diamin, das wenigstens einen cycloaliphatischen Rest aufweist, und
ii) Dialkylentriamin besteht, und wobei
b) die zweite Komponente ein Diamin oder ein Triamin ist, das ausgewählt ist aus der Gruppe, die aus
i) Diamin, das wenigstens einen cycloaliphatischen Rest aufweist,
ii) Dialkylentriamin,
iii) Alkylendiamin und
iv) Oxyalkylendiamin besteht, und
wobei die erste Komponente von der zweiten Komponente verschieden ist.

4. Verwendung gemäß mindestens einem der Ansprüche 1 bis 3, **dadurch gekennzeichnet, dass** der polymere oder oligomere Wirkstoff ein Iminoimidazolderivat ist.

5. Verwendung gemäß mindestens einem der Ansprüche 1 bis 4, **dadurch gekennzeichnet, dass** der polymere oder oligomere Wirkstoff eine Struktur aufweist, die aus der Gruppe umfassend 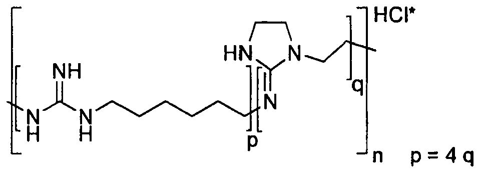 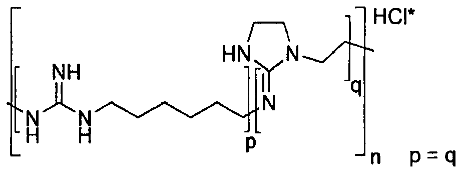 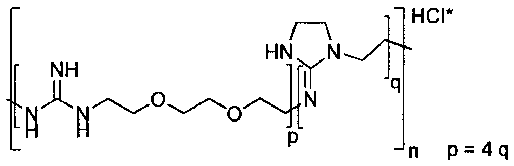 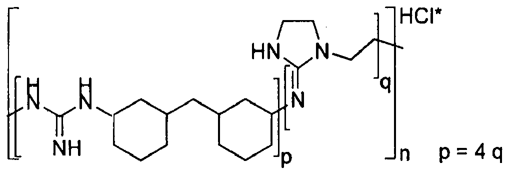 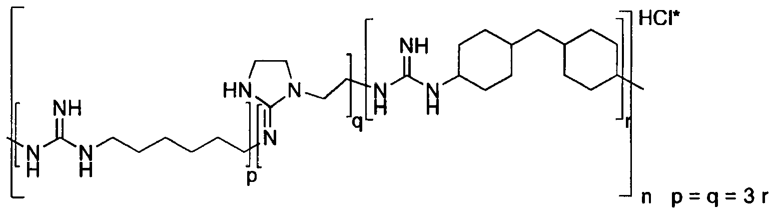 ausgewählt ist, wobei
HCl* bedeutet, dass das HCl nicht kovalent gebunden ist,
n ist eine natürliche Zahl, vorzugsweise von 1 bis 20, weiter bevorzugt von 2 bis 16 und insbesondere von 3 bis 8,
p, q und r sind ganze Zahlen, die das bevorzugte molare Verhältnis der Strukturfragmente zueinander in den Formeln definieren.

6. Verwendung gemäß mindestens einem der Ansprüche 1 bis 5, **dadurch gekennzeichnet, dass** der polymere oder oligomere Wirkstoff ein mittleres Molekulargewicht im Bereich von 500 bis 7000, insbesondere von 1000 bis 5000 aufweist.

7. Verwendung gemäß mindestens einem der Ansprüche 1 bis 6, **dadurch gekennzeichnet, dass** das polymere Guainidinderivat in einer Menge von maximal 10,0 Gew.-%, insbesondere von 0,01 bis 5 Gew.-% und im Speziellen in einer Menge von 1,0 bis 4,0 Gew.-%, bezogen auf die Zusammensetzung für den medizinischen Artikel, vorliegt.

8. Verwendung gemäß mindestens einem der Ansprüche 1 bis 7, **dadurch gekennzeichnet, dass** die Zusammensetzung weiterhin mindestens einen Kunststoff, vorzugsweise mindestens ein thermoplastisches Polymer, insbesondere ausgewählt aus Polyurethan, Polyolefin, Polyvinylchlorid, Polycarbonat, Polystyrol, Polyethersulfon, Silikon und Polyamid aufweist.

9. Verwendung gemäß mindestens einem der Ansprüche 1 bis 8, **dadurch gekennzeichnet dass** der medizinische Artikel ausgewählt ist aus der Gruppe bestehend aus zentralvenösen Kathetern; peripheren Venenkathetern; Beatmungsschläuche; Stents; Produkten für Anwendung in der Regionalanästhesie, insbesondere Katheter, Kuppelungen, Filter; Produkten für die Infusionstherapie, insbesondere Behältnisse, Ports, Überleitungssysteme, Filter; Zubehör wie Konnektoren, Spikes, Ventile, Dreiwegehähne, Spritzen, Leitungen, Zuspritzports; Produkte der Zubereitung, insbesondere Transfersets, Mixsets; urologische Produkte, insbesondere Katheter, Urinmess- und -sammelgeräte; Wunddrainage; Wundversorgung; chirurgische Nahtmaterialien; Implantatzubehörteile und Implantate, insbesondere Kunstoffimplantate, beispielsweise Herniennetze, Vliese, Gestricke, Gewirke, Ports, Portkatheter, Gefäßprothesen; Desinfektionsmittel; chirurgisches Einmalinstrumentarium; Thoraxdrainagen; Sonden; Katheter; Gehäuse von medizinischen Geräten, insbesondere Infusionspumpen, Dialysegeräte und Monitore, künstliche Gebisse; Behälter für Flüssigkeiten, insbesondere Kontaktlinsenbehälter; Reinigungsmittel und Desinfektionsmittel.

10. Verfahren zur Herstellung eines medizinischen Artikels, umfassend die folgenden Schritte
a) Zusammenführen und Vermischen eines polymeren oder oligomeren Wirkstoffs mit biozider Wirkung, welches erhältlich ist durch Polykondensation eines Guanidin-Säureadditionssalzes mit einem Amingemisch, welches wenigstens ein Diamin und/oder ein Triamin enthält, wobei wenigstens ein Amin ausgewählt ist aus der Gruppe, die aus
i) Diamin, welches mindestens einen cycloaliphatischen Rest aufweist, und
ii) Dialkylentriamin
besteht, mit mindestens einem Kunststoff, insbesondere einem thermoplastischen Polymer
b) Einsetzen des unter a) erhaltenen Gemisches in einem oder mehreren Formgebungsverfahren unter Ausbildung eines medizinischen Artikels.

11. Verfahren gemäß Anspruch 10, **dadurch gekennzeichnet, dass** der polymere oder oligomere Wirkstoff dem Formgebungsverfahren in Schritt b) als Granulat oder Masterbatch zugeführt wird.

12. Verfahren gemäß Anspruch 10 oder 11, **dadurch gekennzeichnet, dass** das Vermischen in Schritt a) in einem Extruder erfolgt.

13. Verfahren gemäß mindestens einem der Ansprüche 10 bis 12, **dadurch gekennzeichnet, dass** das Formgebungsverfahren eine Extrusion ist.

14. Medizinischer Artikel umfassend einen polymeren oder oligomeren Wirkstoff mit biozider Wirkung, welches erhältlich ist durch Polykondensation eines Guanidin-Säureadditionssalzes mit einem Amingemisch, welches wenigstens ein Diamin und/oder ein Triamin enthält, wobei wenigstens ein Amin ausgewählt ist aus der Gruppe, die aus
i) Diamin, das wenigstens einen cycloaliphatischen Rest aufweist, und
ii) Dialkylentriamin besteht,
sowie mindestens einen Kunststoff.

15. Medizinischer Artikel gemäß Anspruch 14, **dadurch gekennzeichnet, dass** es ein schlauchförmiger medizinischer Artikel, insbesondere ein Katheter ist.

## Claims

1. Use of a polymeric or oligomeric active ingredient having biocidal activity that is obtainable by the polycondensation of a guanidine acid addition salt with a mixture of amines containing at least one diamine and/or triamine, wherein at least one amine is selected from the group consisting of
i) a diamine having at least one cycloaliphatic residue; and
ii) dialkylene triamine
as an additive in a composition for medical articles.

2. The use according to claim 1, **characterized in that** said mixture of amines comprises the diamine 4,4'-methylenebis(cyclohexylamine).

3. The use according to at least one of claims 1 or 2, **characterized in that** said mixture of amines contains a first component and at least one second component, wherein
a) the first component is a diamine or triamine selected from the group consisting of
i) a diamine having at least one cycloaliphatic residue; and
ii) dialkylene triamine, and wherein
b) the second component is a diamine or triamine selected from the group consisting of
i) a diamine having at least one cycloaliphatic residue;
ii) dialkylene triamine;
iii) alkylene diamine; and
iv) oxyalkylene diamine; and
wherein the first component is different from the second component.

4. The use according to at least one of claims 1 to 3, **characterized in that** said polymeric or oligomeric active ingredient is an iminoimidazole derivative.

5. The use according to at least one of claims 1 to 4, **characterized in that** said polymeric or oligomeric active ingredient has a structure selected from the group comprising 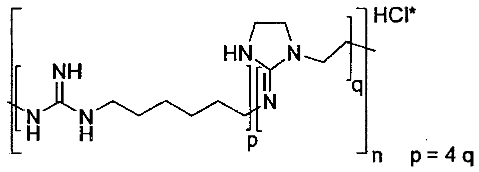 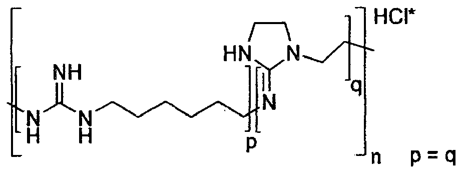 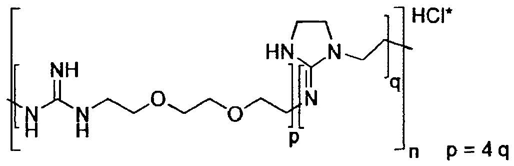 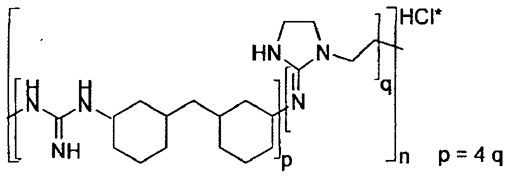 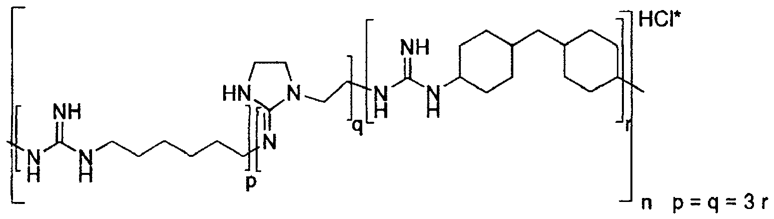 wherein
HCl* means that the HCl is not covalently bonded,
n is a natural number, preferably from 1 to 20, more preferably from 2 to 16, especially from 3 to 8,
p, q and r are integers defining the preferred molar ratio of the structural fragments in the formulas.

6. The use according to at least one of claims 1 to 5, **characterized in that** said polymeric or oligomeric active ingredient has an average molecular weight within a range of from 500 to 7000, especially from 1000 to 5000.

7. The use according to at least one of claims 1 to 6, **characterized in that** said polymeric guanidine derivative is present in an amount of at most 10.0% by weight, particularly from 0.01 to 5% by weight, and especially in an amount of from 1.0 to 4.0% by weight, based on the composition for the medical article.

8. The use according to at least one of claims 1 to 7, **characterized in that** said composition further includes at least one plastic material, preferably at least one thermoplastic polymer, especially selected from polyurethane, polyolefin, polyvinyl chloride, polycarbonate, polystyrene, polyethersulfone, silicone and polyamide.

9. The use according to at least one of claims 1 to 8, **characterized in that** said medical article is selected from the group consisting of central venous catheters; peripheral venous catheters; breathing tubes, stents; products for application in regional anesthesia, especially catheters, couplings, filters; products for infusion therapy, especially containers, ports, conduit systems, filters; accessories, such as connectors, spikes, valves, three-way stopcocks, syringes, conduits, injection ports; products of formulation, especially transfer sets, mixing sets; urological products, especially catheters, urine measuring and collecting devices; wound drains; wound dressing; surgical suture materials; implantation auxiliaries as well as implants, especially plastic implants, for example, hernia meshes, non-wovens, knitwear/knitted fabrics, ports, port catheters, vascular prostheses; disinfectants; disposable surgical instruments; thoracic drains; probes; catheters; housings of medical devices, especially infusion pumps, dialysis devices and screens; artificial dentures; containers for liquids, especially contact lens containers; cleaning agents and disinfectants.

10. A process for preparing a medical article, comprising the following steps:
a) combining and mixing a polymeric or oligomeric active ingredient having biocidal activity that is obtainable by the polycondensation of a guanidine acid addition salt with a mixture of amines containing at least one diamine and/or triamine, wherein at least one amine is selected from the group consisting of
i) a diamine having at least one cycloaliphatic residue; and
ii) dialkylene triamine
with at least one plastic material, preferably a thermoplastic polymer;
b) subjecting the mixture obtained under a) to one or more shaping methods to form a medical article.

11. The process according to claim 10, **characterized in that** said polymeric or oligomeric active ingredient is subjected to the shaping method in step b) as pellets or as a master batch.

12. The process according to claim 10 or 11, **characterized in that** said mixing in step a) is performed in an extruder.

13. The process according to at least one of claims 10 to 12, **characterized in that** said shaping method is an extrusion.

14. A medical article comprising a polymeric or oligomeric active ingredient having biocidal activity that is obtainable by the polycondensation of a guanidine acid addition salt with a mixture of amines containing at least one diamine and/or triamine, wherein at least one amine is selected from the group consisting of
i) a diamine having at least one cycloaliphatic residue; and
ii) dialkylene triamine,
and at least one plastic material.

15. The medical article according to claim 14, **characterized by** being a tubular medical article, especially a catheter.

## Revendications

1. Utilisation d'un principe actif polymère ou oligomère à effet biocide qui peut être obtenu par la polycondensation d'un sel d'addition acide de guanidine avec un mélange d'amines contenant au moins une diamine et/ou une triamine, au moins une amine étant choisie dans le groupe consistant en
i) une diamine présentant au moins un reste cycloaliphatique, et
ii) la dialkylène triamine,
en tant qu'additif dans une composition destinée à des articles médicaux.

2. Utilisation selon la revendication 1, **caractérisée en ce que** ledit mélange d'amines comprend la diamine 4,4'-méthylènebis(cyclohexylamine).

3. Utilisation selon au moins une des revendications 1 ou 2, **caractérisée en ce que** ledit mélange d'amines contient un premier composant et au moins un second composant, dans laquelle
a) ledit premier composant est une diamine ou triamine choisie dans le groupe consistant en
i) une diamine présentant au moins un reste cycloaliphatique, et
ii) une dialkylène triamine, et dans laquelle
b) ledit second composant est une diamine ou triamine choisie dans le groupe consistant en
i) une diamine présentant au moins un reste cycloaliphatique,
ii) une dialkylène triamine,
iii) une alkylène diamine et
iv) une oxyalkylène diamine, et
dans laquelle ledit premier composant est différent dudit second composant.

4. Utilisation selon au moins une des revendications 1 à 3, **caractérisée en ce que** ledit principe actif polymère ou oligomère est un dérivé d'iminoimidazole.

5. Utilisation selon au moins une des revendications 1 à 4, **caractérisée en ce que** ledit principe actif polymère ou oligomère présente une structure choisie dans le groupe comprenant 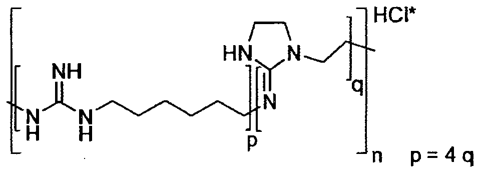 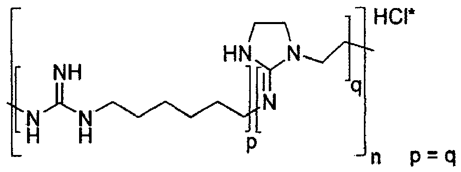 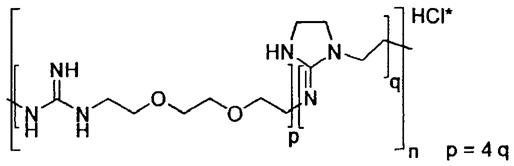 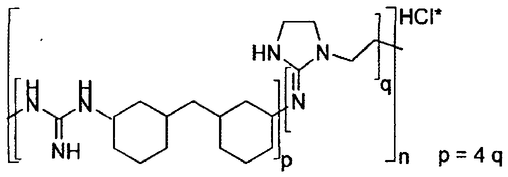 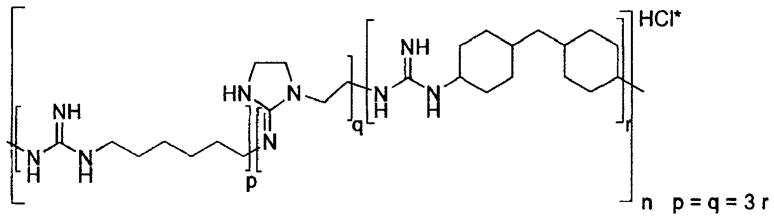 où
HCl* signifie que le HCl n'est pas lié de manière covalente,
n est un entier naturel, de préférence de 1 à 20, plus préférablement de 2 à 16, notamment de 3 à 8,
p, q et r sont des entiers définissant le rapport molaire mutuel préféré des fragments structuraux dans les formules.

6. Utilisation selon au moins une des revendications 1 à 5, **caractérisée en ce que** ledit principe actif polymère ou oligomère présente un poids moléculaire moyen compris entre 500 et 7000, notamment entre 1000 et 5000.

7. Utilisation selon au moins une des revendications 1 à 6, **caractérisée en ce que** ledit dérivé polymère de guanidine est présent en une quantité d'au plus 10,0 % en poids, notamment de 0,01 à 5 % en poids, et spécifiquement en une quantité de 1,0 à 4,0 % en poids, par rapport à ladite composition destinée audit article médical.

8. Utilisation selon au moins une des revendications 1 à 7, **caractérisée en ce que** ladite composition comprend en outre au moins une matière plastique, de préférence au moins un polymère thermoplastique, notamment choisi parmi polyuréthane, polyoléfine, polychlorure de vinyle, polycarbonate, polystyrène, polyéthersulfone, silicone et polyamide.

9. Utilisation selon au moins une des revendications 1 à 8, **caractérisée en ce que** ledit article médical est choisi dans le groupe consistant en des cathéters veineux central, des cathéters veineux périphérique, des tubes respiratoires, des endoprothèses, des produits destinés à être appliqués dans l'anesthésie régionale, notamment des cathéters, des couplages, des filtres ; des produits destinés à la thérapie par infusion, notamment des récipients, des prises, des systèmes de transition, des filtres ; des accessoires, comme des connecteurs, des aiguilles, des valves, des robinets à trois voies, des seringues, des conduits, des ouvertures à injection ; des produits de formulation, notamment des ensembles de transfert, des ensembles de mixage ; des produits urologiques, notamment des cathéters, des dispositifs de mesure et de collection d'urine ; des drains de plaie ; des pansements pour plaies ; des matériaux de suture chirurgicale ; des accessoires d'implantation et des implants, notamment des implants en matière plastique, par exemple, des bandages herniaires, des non-tissés, des tricots, des tissus de mailles, des prises, des cathéters à chambre implantables, des prothèses vasculaires ; des désinfectants ; des instruments chirurgicaux jetables ; des drains thoraciques ; des sondes ; des cathéters ; des boîtiers pour dispositifs médicaux, notamment pour les pompes à infusion, les dispositifs de dialyse et les écrans, des dentures artificielles ; des récipients pour liquides, notamment des étuis pour lentilles de contact ; des produits de nettoyage et des désinfectants.

10. Procédé pour la production d'un article médical, comprenant les étapes consistant à :
a) combiner et mélanger un principe actif polymère ou oligomère à effet biocide qui peut être obtenu par la polycondensation d'un sel d'addition acide de guanidine avec un mélange d'amines contenant au moins une diamine et/ou une triamine, au moins une amine étant choisie dans le groupe consistant en
i) une diamine présentant au moins un reste cycloaliphatique, et
ii) la dialkylène triamine,
avec au moins une matière plastique, notamment un polymère thermoplastique ;
b) soumettre le mélange obtenu dans l'étape a) dans un ou plusieurs procédés de façonnage pour former un article médical.

11. Procédé selon la revendication 10, **caractérisé en ce que** ledit principe actif polymère ou oligomère est soumis audit procédé de façonnage dans l'étape b) sous forme de granules ou de mélange-maître.

12. Procédé selon la revendication 10 ou 11, **caractérisé en ce que** le mélange dans l'étape a) est effectué dans une extrudeuse.

13. Procédé selon au moins une des revendications 10 à 12, **caractérisé en ce que** ledit procédé de façonnage est une extrusion.

14. Article médical, comprenant un principe actif polymère ou oligomère à effet biocide qui peut être obtenu par la polycondensation d'un sel d'addition acide de guanidine avec un mélange d'amines contenant au moins une diamine et/ou une triamine, au moins une amine étant choisie dans le groupe consistant en
i) une diamine présentant au moins un reste cycloaliphatique, et
ii) la dialkylène triamine,
et au moins une matière plastique.

15. Article médical selon la revendication 14, **caractérisé en ce qu'**il s'agit d'un article médical tubulaire, notamment d'un cathéter.
